# EUROPEAN PATENT APPLICATION

(11) **EP 2 172 206 A1**
(43) Date of publication of application: **07.04.2010**
(21) Application number: 08748683.3
(22) Date of filing: 28.05.2008
(51) Int. Cl.: A61K 36/48, A61K 36/16, A61P 3/10, A61P 9/00, A61P 9/10, A61P 25/00, A61P 27/02, A61P 13/12, A61K 133/00

(54) **THE METHOD FOR A SEQUOYITOL-CONTAINING EXTRACT OBTAINING FROM THE GENUS OF TRIFOLIUM, SOBYEAN AND GINKGO BILOBA AND USE THEREOF**

(30) Priority: 30.05.2007 CN 200710028333
(71) Applicant: Lianchuangsiyuanli Biology And Science And Technology Co. Ltd., Guangdong 510075 (CN)
(72) Inventor: SUN, Tianyu, Guangzhou Guangdong 510075 (CN); WANG, Yanli, Guangzhou Guangdong 510075 (CN); DENG, Guixing, Guangzhou Guangdong 510075 (CN); WANG, Yinyin, Guangzhou Guangdong 510075 (CN); LU, You, Guangzhou Guangdong 510075 (CN); WANG, Ting, Guangzhou Guangdong 510075 (CN)
(74) Representative: Pulieri, Gianluca Antonio
(86) International application number: PCT/CN2008/071118
(87) International publication number: WO 2008/145064

(57) **Abstract**

The method for obtaining a sequoyitol-containing extract from a plant of the genus of Trifolium, Leguminosae or Ginkgoaceae, an extract obtained by the method and the use of the extract for the preparation of medicaments for treating diabetes and its complications, the method comprises the steps of extracting with a solvent, recovering the solvents to give an extractum, subjecting the extractum to two-phase extraction and column chromatography, collecting the fractions containing sequoyitol, concentrating, filtration and drying.

## Description

The application claims the priority of Chinese patent application for invention No. 200710028333.5 entitled "a method for obtaining the extract from several plants and the use of the extract", filed on May 30, 2007 with the State Intellectual Property Office of the People's Republic of China, which is incorporated herein by reference in its entirety.

### FIELD OF INVENTION

The present invention relates to a method for obtaining a sequoyitol-containing extract and the use of the extract. In particular, the present invention relates to a method for obtaining a sequoyitol-containing extract from a plant of the Genus of Trifolium, Leguminosae or Ginkgoaceae, the sequoyitol-containing extract obtained by the method and the use of the extract.

### BACKGROUND OF THE INVENTION

Diabetes (called "xiaoke disease" in traditional Chinese medicine) is a common disease in clinic. Diabetes has become a worldwide problem due to its complicated pathopoiesis, multiple complications and low cure rate. Diabetes is ranked as the second leading cancer by the World Health Organization (WHO). In China, there are about eighty million patients suffering from diabetes, with complications that may cause disability, or even are life-threatening.

Diabetes is a metabolic disease characterized by high levels of blood glucose. It may cause the vascular diseases of the heart, brain, kidney, lower limbs and the like, and may lead to severe consequences, such as hypertension, heart disease, diabetic nephropathy and the like. The pathogenic mechanism of diabetes has not been well understood by now, and the majority of therapies of diabetes are focused on lowering the high level of blood glucose and the prophylaxis of complications. Currently used drugs have the disadvantage of strong side effects, and insulin is too expensive for ordinary families. Thus, there is a great need for a drug from plants that treats diabetes with high safety and efficiency as well as low cost.

It was reported that inositols, such as myo-inositol and D-Chiro-inositol, play an important role in the signaling of insulin's effects. The results from animal experiments indicated that inositols may promote the function of insulin and lower the levels of blood glucose, blood insulin and blood triglycerides and the like. Moreover, it also has a protective effect for diabetic retinal pericapillary cells. The methyl derivatives of inositols also have the effect of decreasing blood glucose.

The present invention provides sequoyitol extracted from plants, which is a myo-inositol derivative. Pharmacological experiments show that it presents prophylactic and therapeutic effects on diabetes.

Chinese patent application for invention No. 02144302.5 by Liang Jingyu et al. discloses that the sequoyitol isolated from *Taxus chinensis* is an antidiabetes drug with very low toxicity that remarkably decreases high glycemia in diabetes models, inhibits the breakdown of hepatic glycogen and the absorption of glucose. However, *Taxus chinensis* is under plant protection and there are no abundant sources available. Accordingly, it is expensive to extract sequoyitol from *Taxus chinensis.*

### SUMMARY OF THE INVENTION

An object of the present invention is to provide a method for obtaining a sequoyitol-containing extract from a plant of the genus of Trifolium, Leguminosae or Ginkgoaceae.

Another object of the present invention is to provide a sequoyitol-containing extract prepared by the above-mentioned method.

A further object of the present invention is to provide the use of the sequoyitol-containing extract or pharmaceutically acceptable salts thereof for the preparation of a medicament for treating diabetes and its complications.

The present invention relates to a method for obtaining a sequoyitol-containing extract from a plant of the genus of Trifolium, Leguminosae or Ginkgo, comprising the steps of extracting with a solvent, recovering the solvent to give an extractum, subjecting the extractum to two-phase extraction and column chromatography, collecting the fractions containing sequoyitol, concentration, filtration and drying.

Preferably, the plant of the genus of Trifolium is selected from the group consisting of *Trifolium pretense Linn*., *Trifolium repens Linn., Trifolium incarnatum Linn., Trifolium alexandrinum Linn., Trifolium camperstre Linn., Trifolium dubium Linn., Trifolium eximium Linn., Trifolium fragiferum Linn*., *Trifolium gordejevi Linn*., *Trifolium hybridum Linn., Trifolium lupinaster Linn*. *var*. *albiflorum, Trifolium lupinaster Linn. var. lupinaster, Trifolium medium Linn., Trifolium strepens Linn*., *Trifolium lupinaster Linn.,* and any combination thereof. The plant of the genus of soybean is selected from the group consisting of *Glycine max (Linn.) Merr., Glycine gracilis Skv. Var. gracilis, Glycine clandestine Wendl., Glycine soja Sieb. Et Zucc. Var. albiflora f. angustifolia P. Y Fu et Y A. Chen, Glycine tabacina Benth, Glycine gracilis Skv., Glycine tomentella Hayata, Glycine soja Sieb. et. Zucc. Var albiflora P. Y Fu et Y A. Chen, Glycine gracilis Skv. Var. nigra Skv., Glycine soja Sieb. et. Zucc. Var. soja, Glycine soja Sieb. et. Zucc*., and any combination thereof. The plant of the genus of Ginkgoaceae is selected from the group consisting of *Ginkgo biloba Linn. cv. Biloba, Ginkgo biloba Linn., Ginkgo biloba Linn. cv. Yaweiyinxing, Ginkgo biloba Linn. cv. Xiaofoshou, Ginkgo biloba Linn. cv. Wuxinyinxing, Ginkgo biloba Linn. cv. Tongziguo, Ginkgo biloba Linn. cv. Mianhuaguo, Ginkgo biloba Linn. cv. Luanguofoshou, Ginkgo biloba Linn. cv. Ganglanfoshou, Ginkgo biloba Linn. cv. Fozhi, Ginkgo biloba Linn. cv. Dongtinghuang, Ginkgo biloba Linn. cv. Dameihai, Ginkgo biloba Linn. cv. Damaling,* and any combination thereof.

Preferably, the solvent used in the extraction step is selected from the group consisting of ethanol, methanol, acetone, water and any combinations thereof. Preferably, the temperature of the extraction is 0°C to the reflux temperature of the solvent, preferably room temperature to the reflux temperature of the solvent, more preferably the reflux temperature of the solvent. Preferably, the weight to volume ratio of the solvent and the plant of the genus of Trifolium, Leguminosae or Ginkgoaceae is 1:0.5 to 1:15, preferably 1:2 to 1:10, more preferably 1:3 to 1:5. Preferably, the extraction is performed 2-4 times.

Preferably, the solvents used for the two-phase extraction are a water-immiscible organic solvent and water. Preferably, the water-immiscible organic solvent is selected from the group consisting of ethyl acetate, chloroform, dichloromethane, ethyl ether, petroleum ether, n-butanol and any combination thereof. Preferably, the ratio of the water-immiscible organic solvent to water is 1:0.5 to 1:10 (v/v).

Preferably, the column chromatography is macroporus resin column chromatography, sephadex column chromatography, modified sephadex column chromatography, cellulose column chromatography, activated charcoal column chromatography or ion-exchange resin column chromatography.

Preferably, a purification step is performed after the filtration and prior to the drying. Preferably, the purification step is column chromatography. The column chromatography may be macroporus resin column chromatography, sephadex column chromatography, modified sephadex column chromatography, cellulose column chromatography, activated charcoal column chromatography or ion-exchange resin column chromatography.

The present invention further provides the extract prepared from the above-mentioned method for obtaining a sequoyitol-containing extract.

The present invention further provides the use of the extract prepared by the above method or pharmaceutically acceptable salts thereof for the preparation of medicaments for treating diabetes and its complications. Preferably, the pharmaceutically acceptable salts includes the following salts of sequoyitol: citrate, tartrate, acetate, lactate, sodium salt, potassium salt, calcium salt, ammonium salt, magnesium salt, ferrum salt, zinc salt, aluminum salt and any combination thereof. Preferably, the complications include ocular disorders, kidney disorders, neuropathy, coronary heart disease, cerebrovascular disorders or peripheral vascular disorders.

The present method for obtaining a sequoyitol-containing extract from a plant of the genus of Trifolium, Leguminosae or Ginkgoaceae puts forward an optimized extraction process according to the inherit characteristic of plants of the genus of Trifolium, Leguminosae or Ginkgo, and presents a strategy for industrial production. The sequoyitol-containing extract obtained by the method shows an antidiabetic activity and low toxicity. The major active ingredient of the extract is sequoyitol, the content of which is between 10.00% and 99.99%. Animal experiments shows that the extract could remarkably decrease the level of blood glucose.

### BRIEF DESCRIPTION OF THE DRAWINGS

Figure. 1 shows the Electrospray Ionization Mass Spectrometry ( ESI-MS ) of the sequoyitol in Example 10;

Figure.2 shows the Proton Nuclear Magnetic Resonance Spectroscopy ( ¹H NMR, 400MHz, CD₃OD ) of the sequoyitol in Example 10;

Figure.3 shows the Proton Nuclear Magnetic Resonance Spectroscopy ( ¹H NMR, 400MHz, CD₃OD ) of the sequoyitol in Example 10;

Figure.4 shows the Carbon Nuclear Magnetic Resonance Spectroscopy ( ¹³C NMR, 100MHz, CD₃OD ) of the sequoyitol in Example 10;

Figure.5 shows the retention time plot of sequoyitol determined in the High Performance Liquid Chromatography in Example 11.

### DESCRIPTION OF THE EMBODIMENTS

A detailed description of the technical solution of the present invention is as follows.

The present invention provides a method for extracting and isolating a sequoyitol-containing extract with an antidiabetic activity and low toxicity from a plant of the genus of Trifolium, Leguminosae or Ginkgoaceae. Preferably, the plant of the genus of Trifolium is selected from the group consisting of *Trifolium pretense Linn.(T. pretense), Trifolium repens Linn.(T. repens), Trifolium incarnatum Linn. (T. incarnatum), Trifolium alexandrinum Linn. (T. alexandrinum), Trifolium camperstre Linn. (T. camperstre), Trifolium dubium Linn.(T. dubium), Trifolium eximium Linn.(T. eximium), Trifolium fragiferum Linn. (T. fragiferum), Trifolium gordejevi Linn.(T. gordejevi), Trifolium hybridum Linn. (T. hybridum), Trifolium lupinaster Linn. var. albiflorum(T. lupinaster var. albiflorum), Trifolium lupinaster Linn. var. lupinaster(T. lupinaster var. lupinaster), Trifolium medium Linn. (T. medium), Trifolium strepens Linn. (T. strepens), Trifolium lupinaster Linn.(T. lupinaster)* etc. The plant of the genus of Leguminosae is selected from the group consisting of *Glycine max (Linn.) Merr.), Glycine gracilis Skv. Var. gracilis, Glycine clandestine Wendl., Glycine soja Sieb. Et Zucc. Var. albiflora f. angustifolia P. Y. Fu et Y. A. Chen, Glycine tabacina Benth, Glycine gracilis Skv., Glycine tomentella Hayata, Glycine soja Sieb. et. Zucc. Var albiflora P. Y Fu et Y. A. Chen, Glycine gracilis Skv. Var. nigra Skv., Glycine soja Sieb. et. Zucc. Var. soja, Glycine soja Sieb. et. Zucc.,* etc.

The present invention relates to a method for obtaining a sequoyitol-containing extract from a plant of the genus of Trifolium, Leguminosae or Ginkgoaceae, comprising the steps of extracting with a solvent, recovering the solvents to give an extractum, subjecting the extractum to two-phase extraction and column chromatography, collecting the fractions containing sequoyitol, concentration, filtration and drying. The sequoyitol-containing extract obtained by the method is usually a crystalline powder, in which the content of sequoyitol may be up to 99% or more.

The sequoyitol-containing extract obtained by the above method may be used for the preparation of medicaments and pharmaceutical compositions for treating diabetes and its complications. The sequoyitol-containing extract may be used to prepare injections, capsules, tablets, granules, dragees, solutions etc., or controlled release preparations or targeting preparations of the above dosage forms, in combination with one or more auxiliaries and/or excipients.

Sequoyitol, as well as its pharmaceutically acceptable salts obtained by reacting sequoyitol with respective acids or bases, may be used in the preparation of the formulations of the invention.

The sequoyitol-containing extract obtained by the present method may be used for the preparation of medicaments for treating diabetes and its complications, may markedly decrease diabetic hyperglycemia, inhibit the breakdown of hepatic glycogen and the absorption of glucose, decrease the level of blood fat, improve the metabolism of free radicals and protect islet beta-cells, while with low toxicity. The sequoyitol-containing extract obtained by the present method may also be used for the prevention and treatment of diabetes and its complications, of other diseases associated with glycometabolism disorder, of type II diabetes and its complications, and for the improvement of the metabolism of free radicals.

In particular, the present method for obtaining a sequoyitol-containing extract from a plant of the genus of Trifolium, Leguminosae or Ginkgoaceae generally comprises the following steps. The parts of a plant of the genus of Trifolium, Leguminosae or Ginkgoaceae (such as branches and leaves etc.) are dried in the air and smashed, and then extracted with a solvent such as ethanol, methanol, acetone, or the hydrate or mixtures thereof etc. at a temperature of 0°C to the reflux temperature, preferably room temperature to the reflux temperature, more preferably the reflux temperature. Preferably, the amount of the solvent is 1:0.5 to 15 (w/v), preferably 1:2 to 10 (w/v), more preferably 1:3 to 5 (w/v). Preferably, the extraction is performed 2-4 times. The resulting extractum is concentrated under reduced pressure, then subjected to two-phase extraction with water-immiscible organic solvents (such as ethyl acetate, chloroform, dichloromethane, ethyl ether, petroleum ether) and water, in which the extraction agent preferably is organic solvent/water of 1:0.5 to 10 (v/v). The organic layer is removed. The aqueous layer is pooled, filtrated, and subjected to column chromatography. The column chromatography may be macroporus resin column chromatography (for example, D101, MN-200 (PUROLITE) and the like), sephadex G column chromatography or modified sephadex column chromatography (for example, sephadex-LH-20 and the like), cellulose column chromatography, activated charcoal column chromatography or ion-exchange resin column chromatography. The elution is performed with corresponding eluent, and the eluate is determined. The fractions containing sequoyitol are collected, concentrated under reduced pressure, and purified by column chromatography. The column chromatography may be macroporus resin column chromatography (for example, D101, MN-200 (PUROLITE) and the like), sephadex G or modified sephadex column chromatography (for example, sephadex-LH-20 and the like), cellulose column chromatography, activated charcoal column chromatography or ion-exchange resin column chromatography. The fractions containing sequoyitol are pooled, concentrated under reduced pressure, allowed to set, and filtrated to give a solid, and then recrystallized from ethanol, methanol, acetone, methyl ethyl ketone, or hydrates or mixtures thereof to give dried powder of plant extracts. The final product is crystalline powder in which the major active ingredient is sequoyitol with purity between 10.00% and about 99.99%.

The following method may be used for determining the eluate in the column chromatography of the inventive method.

Thin layer chromatography: F254 thin layer plate is used. 10µl of the test solution or a control solution (aqueous solution of sequoyitol) are loaded on the thin layer plate respectively, then developed with a developing solvent containing n-butanol, ethyl acetate and water (3.5:6.5:1, in volume), visualized by potassium permanganate. The main spot of the test solution with a Rf value equaling to the control solution indicates a positive reaction.

The identification of the structures of the major active ingredients of the extract is carried out by Mass Spectrometry, Hydrogen NMR Spectrometry and Carbon NMR Spectrometry. See example 10 for reference.

The content of sequoyitol, the major active ingredient, of the extract may be determined using the following High Performance Liquid Chromatography (HPLC):

Assay 1 : About 5mg of extract was precisely weighed and charged into a 10 ml volumetric flask. 0.25 ml of diluted sulfuric acid-acetic anhydride (1:50 (v/v)) is added, heated for 30 minutes in water-bath, then cooled to room temperature. 5 ml of methanol is added, evenly mixed. Then water is added to the scale, and evenly mixed to give the test solution. The conditions of HPLC are as follows:
Column: C18 column, 5µl, 4.6x250mm,
Detection wavelength: 220nm,
Sample volume: 20µl,
Mobile phase: methanol/water (50:50 (v/v)), filtrated by 0.45µm organic film filter and degased before use,
Flow rate: 1.0ml/min.

Assay 2 : About 5mg of extract is precisely weighed and charged into a 10 ml volumetric flask, then dissolved in water to the desired volume, and evenly mixed. The conditions of HPLC are as follows:
Column: amino column, 5µl, 4.6x250mm,
Detector: Evaporative Light Scattering Detector (ELSD),
Temperature of the drift tube: 40°C,
Pressure: 3.5 bar,
Sample volume: 20 µl,
Mobile phase: acetonitrile/water (75:25 (v/v)), filtrated by 0.45µm organic film filter and degased before use,
Flow rate: 0.8ml/min.

In clinic, the extract obtained from *Nephrolepis auriculata* may be used for the preparation of various drugs or health care products etc.

One or more conventional excipients may be comprised in the capsules, tablets, granules, dregees etc. In particular, the excipients may be as follows:
Filler and solubilizer: starch, microcrystalline cellulose, etc.,
Binder: carboxymethylcellulose, polyvinyl pyrrolidone, etc.,
Wetting agent: glycerol, etc.,
Disintegrating agent: calcium carbonate, etc.,
Absorption agent: kaoline, etc.,
Lubricant: talc powder, etc.

The pharmaceutical excipients may be in the form of solid, semi-solid, liquid or other forms. Various types of auxiliaries can be used in formulation.

Solutions can comprise conventional solvents, solubilizers, emulsifiers, preservatives, etc, such as water, ethanol, glycerol, polyethylene glycol, benzylbenzoate, etc.

The dosage forms, such as capsules, tablets, granules, dregees, and solutions, may be prepared by known methods. The plant extracts may be mixed with one or more excipients to prepare the dosage forms.

Formulations containing traditional Chinese medicinal extracts for treating diabetes may be prepared according to the present invention, thus new alternatives for the treatment of diabetes are provided. In addition, the plants of the genus of Trifolium, Leguminosae or Ginkgoaceae are abundant and well available. The present method involves a simplified production process and low cost. A natural medicine which has significant therapeutic effect on diabetes is thereby provided.

The present invention is further described in combination with the following examples.

### Example 1: the preparation of plant extracts with lowest purity-1

100 kg of crude powder of plant (*Trifolium medium Linn.*) was extracted three times under refluxing in 3 volumes of 90% ethanol. The resulting extract solution was pooled, concentrated under reduced pressure, and extracted by a two-phase system of ethyl acetate and water (1:2 (v/v)). The aqueous layers were pooled, filtrated, and subjected to macroporus resin column chromatography (D101). Elution was done with gradients of distilled water and aqueous ethanol. The fractions containing sequoyitol were collected, concentrated under reduced pressure, and dried to give a plant extract (yellowish powder), in which the content of sequoyitol was 10.1%.

### Example 2: the preparation of plant extract with low purity-1

50 kg of crude powder of plants (*Glycine clandestine Wendl.*) was extracted three times under refluxing in 3 volumes of 90% ethanol at 60°C. The resulting extract solution was pooled, concentrated under reduced pressure until dry, and extracted with a two-phase system of dichloromethane and water (1:1 (v/v)). The aqueous layers were combined, filtrated, and subjected to macroporus resin column chromatography (D101). Elution was done with gradients of distilled water and aqueous ethanol (ethanol: 5%-30%). The eluates were detected with detection reagents respectively. The eluates exhibiting positive reactions were collected, concentrated under reduced pressure, and dried at 70°C to give a plant extract (yellowish powder), in which the content of sequoyitol was 27.6%.

### Example 3: the preparation of plant extract with low purity-2

60 kg of crude powder of plants (*Ginkgo biloba Linn. cv. Damaling.*) was extracted three times under refluxing in 3 volumes of 90% methanol at 50°C. The resulting extract solution was combined, concentrated under reduced pressure until dry, and extracted with a two-phase system of ethyl acetate and water (1:1 (v/v)). The aqueous layers were combined, filtrated, and subjected to macroporus resin column chromatography (MN-200). Elution was done with gradients of distilled water and aqueous ethanol (ethanol: 5%-20%). The eluates were detected with detection reagents respectively. The eluates exhibiting positive reactions were collected, concentrated under reduced pressure, and dried at 70°C to give a plant extract (yellowish powder), in which the content of sequoyitol was 28.2%.

### Example 4: the preparation of plant extract of low purity-3

70 kg of crude powder of plants (*Glycine tabacina Benth.*) was extracted three times under refluxing in 3 volumes of 70% acetone at 45°C. The resulting extract solution was combined, concentrated under reduced pressure until dry, and extracted with a two-phase system of chloroform and water (1:1(v/v)). The aqueous layers were combined, filtrated, and subjected to activated charcoal column chromatography. Elution was done with gradients of distilled water and aqueous ethanol (ethanol: 5%-25%). The eluates were detected with detection reagents respectively. The eluates exhibiting positive reactions were collected, concentrated under reduced pressure, and dried at 70°C to give a plant extract (yellowish powder), in which the content of sequoyitol was 30.0%.

### Example 5: the preparation of plant extract with moderate purity-1

The plant extract with low purity was dissolved in water, and subjected to cellulose column chromatography. Elution was done with gradients of distilled water and aqueous ethanol (ethanol: 5%-20%). The eluates were detected with detection reagents respectively. The eluates exhibiting positive reactions were collected, concentrated under reduced pressure , recrystallized in ethanol, and filtrated to give a crystalline powder. The resulting crystalline powder was vacuum dried, and dried at 70°C to give plant extract (yellowish powder), in which the content of sequoyitol was 76.2%, as determined HPLC. The plant extract with low purity in this example was any extract obtained by the methods of examples 1-4.

### Example 6: the preparation of plant extract with moderate purity-2

The plant extract with low purity was dissolved in water, and subjected to ion exchange column chromatography. Elution was done with gradients of distilled water and aqueous ethanol (ethanol: 5%-20%). The eluates were detected with detection reagents respectively. The eluates exhibiting positive reactions were collected, concentrated under reduced pressure until dry, recrystallized in methanol, and filtrated to give a crystalline powder. The resulting crystalline powder was vacuum dried, and dried at 70°C to give plant extract (yellowish powder), in which the content of sequoyitol was 74.6%, as determined by HPLC. The plant extract with low purity in this example was any extract obtained by the methods of examples 1-4.

### Example 7: the preparation of plant extract with moderate purity-3

The plant extract with low purity was dissolved in water, and subjected to gel column chromatography. Elution was done with gradients of distilled water and aqueous ethanol (ethanol: 5%-15%). The eluates were detected with detection reagents respectively. The eluates exhibiting positive reactions were collected, concentrated under reduced pressure until dry, recrystallized in acetone, and filtrated to give a crystalline powder. The resulting crystalline powder was vacuum dried, and dried at 70°C to give a plant extract (yellowish powder), in which the content of sequoyitol was 72.5%, as determined by HPLC. The plant extract with low purity in this example was any extract obtained by the methods of examples 1-4.

### Example 8: comparison of sequoyitol extracts derived from various plants

The methods of the foregoing Examples 1-7 were applied to several plants, and the contents of sequoyitol of the resulted extracts were determined by HPLC (table 1). The values listed under the column "low content" in table 1 are average values after extracting specific crude plant powders according to examples 1-4. The values listed under the column "high content" in table 1 were obtained by the following steps: a) extracting specific crude plant powder by a method randomly selected from examples 1-4, and b) further extracting the resulted extract by a method randomly selected from examples 5-7, obtaining a specific value of the sequoyitol content; the above steps a) and b) were repeated several times, obtaining several values of the sequoyitol content; then the resulted values of the sequoyitol content were averaged, giving the values listed in the column of high content in Table 1.

**Table 1. The content of sequoyitol of the sequoyitol extracts derived from crude powders of different plants**

| Plant | Low content | High content |
|---|---|---|
| T. incarnatum | 27.6% | 73.1% |
| T. repens | 26.7% | 70.0% |
| T. pratense | 31.4% | 79.5% |
| T. camperstre | 28.6% | 69.8% |
| T. alexandrinum | 25.4% | 67.1% |
| T. dubium | 30.7% | 71.5% |
| T. eximium | 34.4% | 74.1% |
| T. fragiferum | 27.5% | 65.9% |
| Ginkgo biloba Linn. cv. Damaling | 29.4% | 69.7% |
| T. gordejevi | 26.1% | 75.9% |
| T. hybridum | 27.7% | 68.4% |
| T. lupinaster var. albiflorum | 28.7% | 70.4% |
| T. lupinaster var. lupinaster | 32.1% | 75.8% |
| T. medium | 27.4% | 76.1% |
| T. strepens | 28.6% | 71.2% |
| T. lupinaster | 26.4% | 68.7% |
| Glycine max (Linn.) Merr. | 27.8% | 75.6% |
| Glycine gracilis Skv. | 28.4% | 74.8% |
| Glycine gracilis Skv. Var. gracilis | 26.9% | 65.8% |
| Glycine gracilis Skv. Var. gracilis | 30.4% | 78.4% |
| Glycine clandestine Wendl. | 31.8% | 75.4% |
| Glycine soja Sieb. Et Zucc. Var. albiflora f. angustifolia | 26.4% | 67.3% |
| P. Y. Fu et Y. A. Chen | | |
| Glycine tabacina Benth. | 31.7% | 76.4% |
| Glycine tomentella Hayata | 29.6% | 70.8% |
| Glycine soja Sieb. et. Zucc. Var albiflora | 26.5% | 71.4% |
| P. Y. Fu et Y. A. Chen | | |
| Glycine soja Sieb. et. Zucc. Var. soja | 29.7% | 69.7% |
| Glycine soja Sieb. et. Zucc. | 29.6% | 68.7% |
| Ginkgo biloba Linn. cv. Mianhuaguo | 27.8% | 70.9% |
| Ginkgo biloba Linn. cv. Tongziguo | 26.7% | 70.4% |
| Ginkgo biloba Linn. cv. Wuxinyinxing | 29.4% | 69.7% |
| Ginkgo biloba Linn. cv. Xiaofoshou | 26.7% | 70.1% |
| Ginkgo biloba Linn. cv. Yaweiyinxing | 27.5% | 72.3% |
| Ginkgo biloba Linn. | 26.8% | 70.4% |
| Ginkgo biloba Linn. cv. Biloba | 27.6% | 71.7% |
| Ginkgo biloba Linn. cv. Dameihai | 30.4% | 73.8% |
| Ginkgo biloba Linn. cv. Dongtinghuang | 31.1% | 76.4% |
| Ginkgo biloba Linn. cv. Fozhi | 29.8% | 72.4% |
| Ginkgo biloba Linn. cv. Ganglanfoshou | 28.5% | 71.4% |
| Ginkgo biloba Linn. cv. Luanguofoshou | 29.9% | 75.2% |
| Glycine gracilis Skv. Var. nigra Skv. | 24.6% | 69.7% |

### Example 9: the preparation of plant extract with high purity

The plant extract with moderate purity was recrystallized several times in methanol and the like, filtrated to give a crystalline powder. The resulting crystalline powder was vacuum dried, and dried at 70°C to give a plant extract with high purity of sequoyitol. After several repeated recrystallization, the purity of sequoyitol was as high as more than 99.9%.

### Example 10: the identification of the structure of the sequoyitol

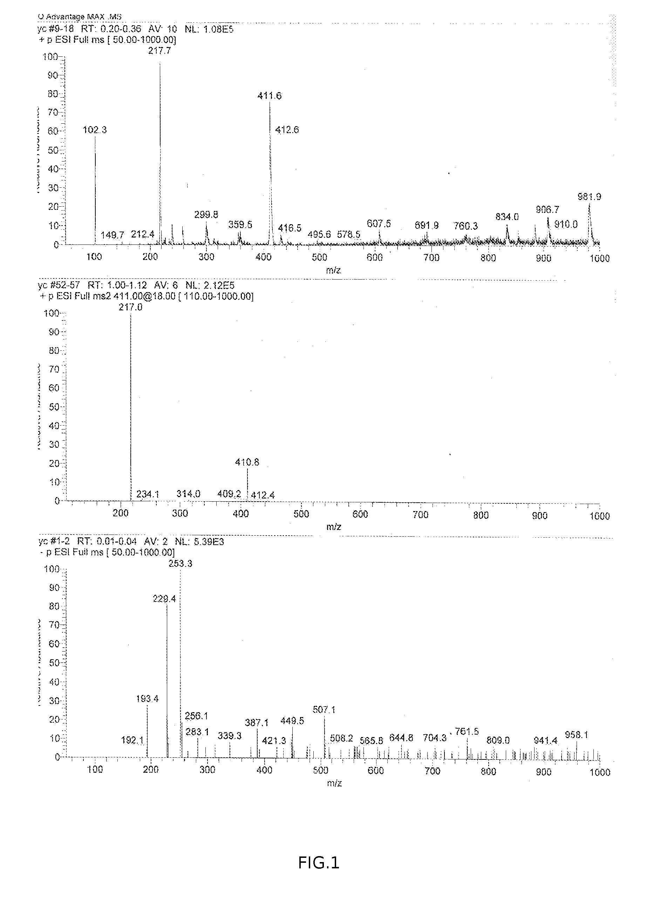

The sequoyitol is a white needle crystal (obtained by repeated recrystallization from methanol) with no ultraviolet absorption (UV spectra). The infrared spectra (IR spectra) indicated hydroxy groups (3347 cm⁻¹). Positive ion ESI-MS results an m/z of 217[M+Na]⁺, while negative ion ESI-MS results an m/z of 193[M-H]⁻ (Figure 1), suggesting that the molecular weight of this compound may be 194. Combining the data of ¹H NMR and ¹C NMR, it is suggested that the molecular formula is C₇H₁₄O₆.

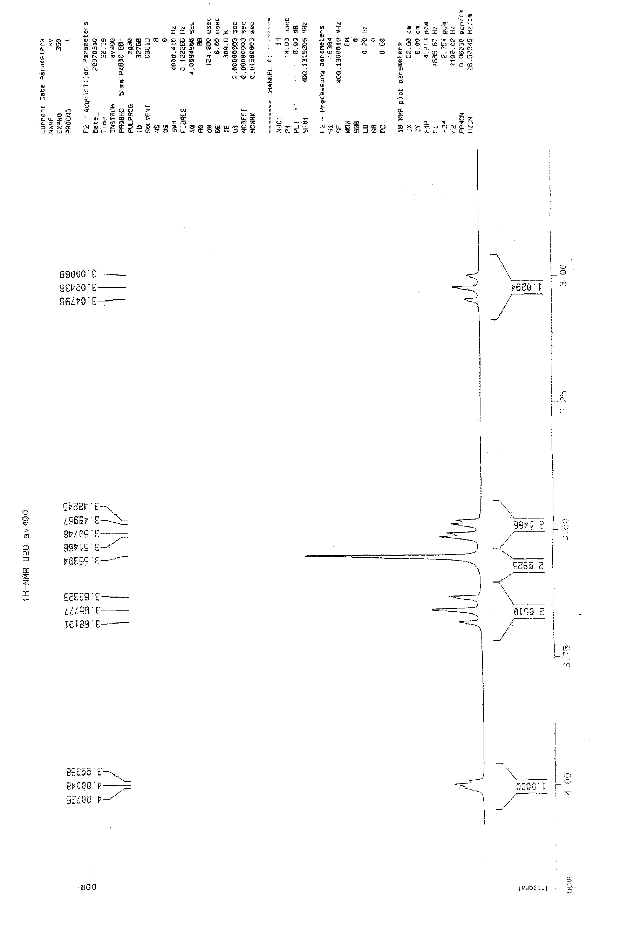

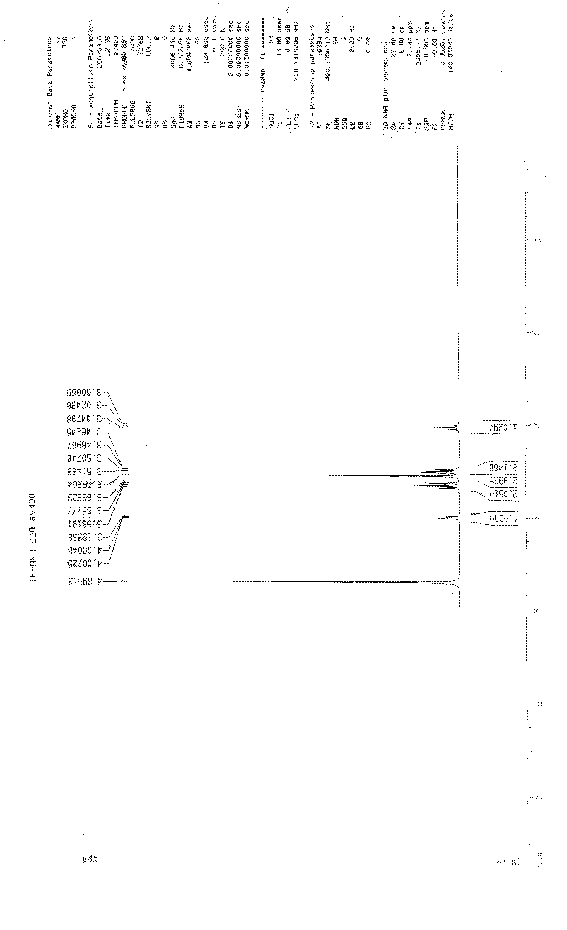

The proton signals shown in ¹H NMR (400MHz, CD₃OD) (Figures 2 and 3) involves the proton signals of one oxygen-linked methyl group ( δ 3.55, s ) and six signals of hydroxy-linked hydrogen [ δ 4.00 ( t, J= 2.84 Hz ), 3.65 ( t, J= 9.82 Hz ), 3.65 ( t, J= 9.82 Hz ), 3.49 ( dd J= 2.87 and 7.12 Hz ), 3.49 ( dd J= 2.87 and 7.12 Hz ) and 3.02 ( t, J= 9.47 Hz )].

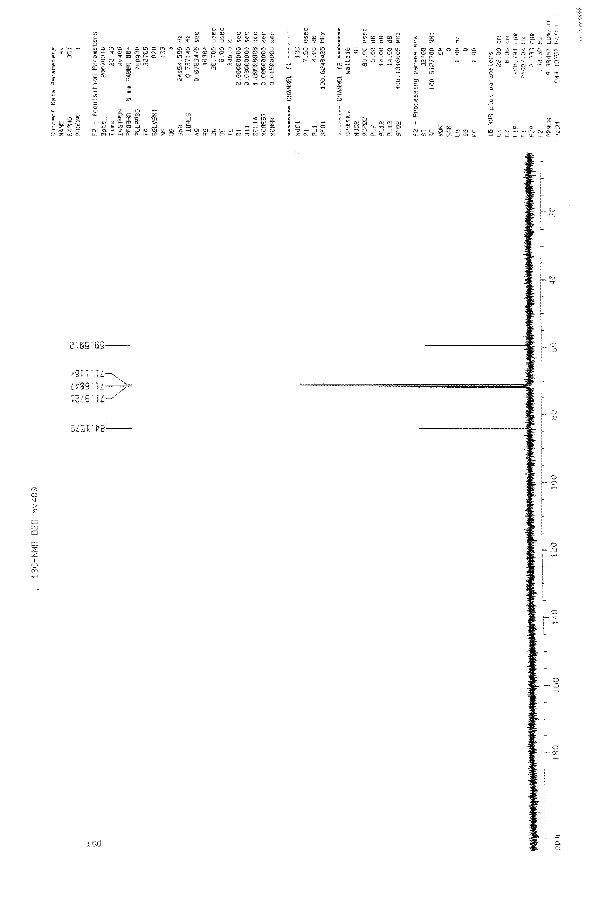

¹³C NMR (100MHz, CD₃OD) (Figure 4) shows that the compound comprises seven carbons, all of which are oxygen-linked carbons. From the above spectra data, it is suggested that the compound may be a cyclitol compound comprising seven carbons.

The ¹H NMR and ¹³C NMR data are in consistent with the data of sequoyitol reported in the Chinese patent application for Invention No. 02144302.5 by Liang jingyu et al., thus it is confirmed that the compound is sequoyitol.

The following structure is obtained from the above analysis on the spectra:

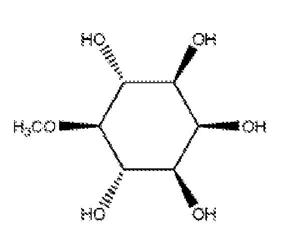

### Example 11: the determination of the content of the sequoyitol extract with high purity

HPLC was used. About 5mg of the extract of high purity was precisely weighed and charged into a 10 ml volumetric flask, then dissolved in water to the desired volume, and mixed envenly. The conditions of HPLC were as follows:
Column: amino column, 5µl, 4.6×250mm,
Detector: Evaporative Light Scattering Detector (ELSD),
Temperature of the drift tube: 40°C,
Pressure: 3.5 bar,
Sample volume: 20 µl,
Mobile phase: acetonitrile/water (75:25 (v/v)), filtrated by 0.45µm organic film filter and degased before use,
Flow rate: 1.0 ml/min.

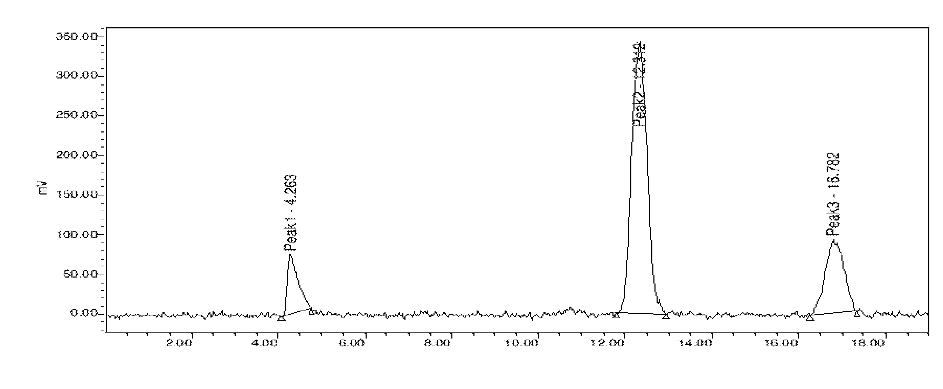

The spectra are shown in figure 5, in which the retention time of sequoyitol is 12.312 min.

### Example 12: the preparation of capsules

Formulation: 1000 capsules were prepared, each capsule weighing 300mg, containing 30mg of plant extract and 270mg of microcrystalline cellulose.

The excipient was placed in a grinder, and then the plant extract was added and grinded for 10∼30 min. The evenly blended mixture was encapsulated into capsules of Size #1. The load of each capsule was 300mg, in which the amount of the plant extract was 30mg.

### Example 13: the stability of the capsules

A 9-month stability test was carried out for 3 batches of capsules, including examinations on the appearance, identification, content and the like.

Identification (1): The contents of 20 capsules were collected. An amount of the powder was taken and dissolved in water, and filtrated. 5ml of the filtrate was placed into a tube. After 2ml of sodium periodate solution (0.01mol/L) and 2 drops of concentrated nitric acid were added, silver nitrate solution was added dropwise, resulting a white precipitate. The results for 3 batches of capsules are shown in Table 2.

Identification (2): 10ml of water was added to an amount of the contents (corresponding to 0.2g of the capsule), shaken up and filtrated. To 2ml of the filtrate, 1ml of basic lead acetate solution was added, mixed evenly. The mixture was heated in water bath for 5 min, obtaining a semitransparent jelly. The results for 3 batches of capsules are shown in Table 2.

**Table 2. The stability of 3 batches of capsules**

| Time | Batch | Appearance | Disintegration | Identification | Identification | Content |
|---|---|---|---|---|---|---|
| (months) | Number | (colour) | time(min) | ( 1 ) | ( 2 ) | ( % ) |
| | 051116 | Off white | ≤ 10 | eligible | eligible | 72.8 |
| 0 | 051117 | Off white | ≤ 10 | eligible | eligible | 69.5 |
| | 051118 | Off white | ≤ 10 | eligible | eligible | 70.6 |
| | 051116 | Off white | ≤ 10 | eligible | eligible | 71.0 |
| 3 | 051117 | Off white | ≤ 10 | eligible | eligible | 70.5 |
| | 051118 | Off white | ≤ 10 | eligible | eligible | 68.9 |
| | 051116 | Off white | ≤ 10 | eligible | eligible | 72.0 |
| 6 | 051117 | Off white | ≤ 10 | eligible | eligible | 68.7 |
| | 051118 | Off white | ≤ 10 | eligible | eligible | 69.1 |
| | 051116 | Off white | ≤ 10 | eligible | eligible | 71.5 |
| 9 | 051117 | Off white | ≤ 10 | eligible | eligible | 70.5 |
| | 051118 | Off white | ≤ 10 | eligible | eligible | 69.3 |

### Example 14: the preparation of tablets

25g of plant extract, 49g of microcrystalline cellulose and 1g of magnesium stearate were well mixed. The resulting mixture was pressed into tablets using single punch tablet press. The tablets were 6 mm in diameter and 300 mg in weight, and each contained 100mg of plant extract.

### Example 15: the preparation of granules

20g of plant extract and 180g of corn starch were well mixed. An appropriate amount of 60% ethanol was added to obtain dough. Then the dough was screened through a 12-mesh sieve to granulate. Granules were obtained after drying. Each gram of granule contained 100mg of plant extract.

### Example 16: the preparation of injections

The refined sequoyitol extract (the purity was 98.00%-99.99%) was used as the starting material of injections.

Sequoyitol are well soluble in water. The sequoyitol was dissolved in water and sterilized by filtration. Solutions for injection were prepared according to the preparation process of solutions for injection. Powder for injection was prepared according to the preparation process of lyophilized powder for injection.

Lyophilized powder for injection was prepared from the refined sequoyitol for injection, according to the preparation process of lyophilized powder for injection. The sequoyitol for injection was dissolved in water for injection. 20% low molecule dextran solution (free of pyrogen) was added. The resulting solution was well mixed. The final concentrations of sequoyitol and dextran were 0.1g/ml and 0.05g/ml respectively. Pyrogen was removed by activated charcoal. The resulted solution was sterilized by a 0.22µm micro filter membrane, and filled into pretreated vials in a clean zone of class 100. Each vial contains 1 ml of solution. In a freeze dryer, the vials were freezed at -40°C for 2 hours, and then sublimated at -25°C under vacuum of 1.33 Pa. After 90% of free water was removed, the vials were dried by heating, with the temperature kept no higher than 35°C. The vials were sealed with stoppers in the freeze dryer.

### Example 17: the preparation of dispersible tablets

60g of dry sequoyitol extract, 20g of microcrystalline cellulose, 10g of cross-linked polyvinylpyrrolidone, 10g of low-substituted hydroxyproxyl cellulose were well mixed. 5% polyvinylpyrrolidone K30 in ethanol was added dropwise to obtain dough. The dough was screened through a 30-mesh sieve to granulate. The wet granules were dried at 60°C, then screened through a 40-mesh sieve. 10g of cross-linked polyvinylpyrrolidone, 10g of low-substituted hydroxyproxyl cellulose and 5g of magnesium stearate were added and well mixed. The resulting mixture was compressed into 1000 tablets.

Determination: (1) Determination of the uniformity of dispersion: 2 dispersible tablets of sequoyitol extract were placed in 100ml water and shaken at 20°C±1°C. They disintegrated completely in 180s and passed through a 2# sieve. (2) The determination of dissolution: the dissolution was determined using artificial gastric fluid (0.1mol/L hydrochloric acid). The determination was carried out according to the second method of the determination of dissolution (paddle) appendixed to the Pharmacopoeia of the People's Republic of China (Part II), at a paddle speed of 100 r/min. The dissolution temperature was 37°C, and the volume of the dissolution medium was 900ml. Dispersible tablets of sequoyitol extract were added into the above dissolution medium under said conditions. 5 ml of aliquots were sampled at 2, 4, 6, 8, 10, 15, 20, 30, 45, and 60 minutes (and supplemented with the same amount of medium), and filtered with 45µm microporous filter membrane. The dissolution was determined, and then relative cumulative dissolution percentages were calculated. The results are shown in Table 3.

**Table 3. The dissolution of dispersible tablets of sequoyitol extract**

| Time (min) | 2 | 4 | 6 | 8 | 10 | 15 | 20 | 30 | 45 | 60 |
|---|---|---|---|---|---|---|---|---|---|---|
| Dissolution (%) | 73.24 | 81.34 | 85.54 | 91.36 | 92.42 | 94.19 | 95.29 | 96.26 | 96.42 | 97.28 |

### Example 18: the preparation of effervescent tablets

Effervescent tablets of sequoyitol extract were prepared according to the following formulation: 60g of sequoyitol extract, 4.2g of tartaric acid, 17.5g of sodium bicarbonate, 1.2g of Tween-80, 3.2g of polyvinyl pyrrolidone(PVP), 1.6g of low-substituted hydroxyproxyl cellulose (LHPC), and 0.64g of magnesium stearate were well mixed and compressed into 1000 tablets. Each tablet contains 60mg of sequoyitol extract.

Examination: 10 test tubes with stopper and graduation (internal diameter: 1.5cm, capacity: 25ml) were filled with precisely 2ml of water respectively, then placed in water bath at 37°C±1°C. 5 minutes later, 1 test tablet was put into each tube, and then seal the test tube with the stopper. The volume of greatest effervescent amount was observed in a period of 20 minutes. The mean effervescent volume was 4.6 ml, with one tablet being less than 3ml.

### Example 19: the preparation of syrup

600g of sucrose was placed in a 1000 ml beaker. 800ml distilled water was added and heated to dissolve the sucrose. 20g of sequoyitol extract was added and dissolved by stirring. The solution was cooled to room temperature and filtrated. The filtrate was collected, and distilled water was added via the filter to 1000ml. After well mixed, the filtrate was aliquoted into 10ml ampoules, and sterilized at 100°C for 30 minutes.

### Example 20: the preparation of delayed release tablets

70g of sequoyitol extract was mixed with 3g of polyvinylpyrrolidone, 9g of sodium alginate and 12g of hydroxypropylmethylcellulose with a mixer (such as high speed shearing granulators or planetary mixers). The mixture was wet granulated. After the resulting granules were dried, 1g of colloidal silicon dioxide and 1g of magnesium stearate were added and mixed. The lubricated granules were compressed into 1000 delayed release tablets, each of which contained 60mg of sequoyitol.

The pharmacodynamic and toxicological tests were performed on the plant extract prepared according to example 1.

### I. Pharmacodynamic test

I). The effect of the plant extract on the elevated level of blood glucose induced by alloxan in mice

### 1. Materials

Animals: 100 male mice weighed 22-25g,
Positive control: Phenformin Hydrochloride (Jiangsu Yabang Pharmaceutical (group) Co., Ltd.),
Alloxan: Sigma.

### 2. Methods

20 mice were randomly selected as the blank group. Otehrs were injected intravenously with Alloxan via caudal vein at a dose of 65mg/kg body weight, and then modeled according to established methods. The mice were divided into 4 groups according to their level of blood glucose, in which 3 groups were administered with 60mg/kg of the plant extract, 75mg/kg of Phenformin Hydrochloride, or 60mg/kg of sequoyitol-containing *Taxus chinensis* extract respectively for 7 days, in a volume of 0.1ml/10g body weight. The same volume of distilled water was administered to the blank group and the control group. Blood glucose was determined using glucose oxidase method.

### 3. Results

The blood glucose levels of the control group were remarkably elevated (by 323.1mg/dl) as compared to the blank group. In comparison to the control group, the hyperglycemia induced by alloxan was remarkably decreased in the 75mg/kg Phenformin Hydrochloride group (by 155.8 mg/dl), the plant extract group (by 120.3 mg/dl), and the sequoyitol-containing *Taxus chinensis* extract group (by 130.3 mg/dl).

### II). The effect of the plant extract on elevated blood glucose level induced by glucose in mice

### 1. Materials

Animals: 120 female mice weighed 22-25g,
Positive control drug: Phenformin Hydrochloride (Jiangsu Yabang Pharmaceutical (group) Co., Ltd.),
50% Glucose Injection: Shandong Lukang Cisen Pharmaceutical Co., Ltd.

### 2. Methods

120 mice were randomly divided into 6 groups, in which 2 groups were administered with 60mg/kg of the plant extract (the content of sequoyitol was 65.7%) for 7 days, in a volume of 0.1ml/10g body weight, while other 2 groups were administered with 60mg/kg of sequoyitol-containing *Taxus chinensis* extract (the content of sequoyitol was 62.3%), in a volume of 0.1ml/10g body weight. The same volume of distilled water was administered to the blank group and control group. The blank group was injected intraperitoneally with physiological saline, while the other groups were injected intraperitoneally with 2g/kg of glucose in the same volume. Blood was collected from the posterior orbital venous plexus at 30, 60, 90 and 120 minutes after injection and the serum was isolated. Blood glucose was determined using glucose oxidase method.

### 3. Results

The blood glucose levels of mice in the control group were remarkbly elevated at 30, 60, 90 and 120 minutes after the injection of glucose, as compared to the blank group. The hyperglycemia induced by the injection of glucose was remarkably decreased by the plant extract and the sequoyitol-containing Taxus chinensis extract at 30, 60, 90 and 120 minutes after the injection of glucose, as compared to the control group. At 90 minutes after the injection, the hyperglycemia was decreased by 14.9mg/dl.

### II. Acute toxicity assay

Method: 40 healthy mice (20 male and 20 female) were administered with 800 mg/kg of *Trifolium pretense* extract and monitored once. 7 days later, the mice were sacrificed, and the organs were examined visually. Results: No fatality was observed. All the mice were healthy, with their skins and hair lustrous and their eyes bright, and showed good motility. After 7 days, all the mice were sacrificed, the major organs were examined visually and no abnormity was found.

The foregoing descriptions merely describe some preferred embodiments of the present invention. It should be noted that various improvements and modifications may be made by those skilled in the art, without departing from the principle of the invention. Thus these improvements and modifications still fall within the scope of the present invention.

## Claims

1. A method for obtaining a sequoyitol-containing extract from a plant of the genus of Trifolium, Leguminosae or Ginkgoaceae, comprising the steps of extracting with a solvent, recovering the solvent to give an extractum, subjecting the extractum to two-phase extraction and column chromatography, collecting the fractions containing sequoyitol, concentrating, filtration and drying.

2. The method for obtaining a sequoyitol-containing extract according to claim 1, **characterized in that** the plant of the genus of Trifolium is selected from the group consisting of *T. pretense, T. repens, T. incarnatum, T. alexandrinum, T. camperstre, T. dubium, T. eximium, T. fragiferum, T. gordejevi, T. hybridum, T. lupinaster var. albiflorum, T. lupinaster var. lupinaster, T. medium, T. strepens, T. lupinaster,* and any combination thereof, wherein:
the plant of the genus of Leguminosae is selected from the group consisting of *Glycine max (Linn.) Merr.), Glycine gracilis Skv. Var. gracilis, Glycine clandestine Wendl., Glycine soja Sieb. Et Zucc. Var. albiflora f. angustifolia P. Y Fu et Y A. Chen, Glycine tabacina Benth, Glycine gracilis Skv., Glycine tomentella Hayata, Glycine soja Sieb. et. Zucc. Var albiflora P. Y Fu et Y A. Chen, Glycine gracilis Skv. Var. nigra Skv., Glycine soja Sieb. et. Zucc. Var. soja, Glycine soja Sieb. et. Zucc*., and any combination thereof, and
the plant of the genus of Ginkgoaceae is selected from the group consisting of *Ginkgo biloba Linn. cv. Biloba, Ginkgo biloba Linn., Ginkgo biloba Linn. cv. Yaweiyinxing, Ginkgo biloba Linn. cv. Xiaofoshou, Ginkgo biloba Linn. cv. Wuxinyinxing, Ginkgo biloba Linn. cv. Tongziguo, Ginkgo biloba Linn. cv. Mianhuaguo, Ginkgo biloba Linn. cv. Luanguofoshou, Ginkgo biloba Linn. cv. Ganglanfoshou, Ginkgo biloba Linn. cv. Fozhi, Ginkgo biloba Linn. cv. Dongtinghuang, Ginkgo biloba Linn. cv. Dameihai, Ginkgo biloba Linn. cv. Damaling*, and any combination thereof.

3. The method for obtaining a sequoyitol-containing extract according to claim 1, **characterized in that** the solvent is selected from the group consisting of ethanol, methanol, acetone, water, and any combination thereof.

4. The method for obtaining a sequoyitol-containing extract according to claim 3, **characterized in that** the temperature of the extraction step is between 0°C and the reflux temperature of the solvent, preferably between room temperature and the reflux temperature of the solvent, more preferably the reflux temperature of the solvent.

5. The method for obtaining a sequoyitol-containing extract according to claim 4, **characterized in that** the weight to volume ratio of the solvent and the plant of the genus of Trifolium, Leguminosae or Ginkgoaceae is 1:0.5 to 15 (w/v), preferably 1:2 to 10 (w/v), more preferably 1:3 to 5 (w/v).

6. The method for obtaining a sequoyitol-containing extract according to claim 1, **characterized in that** the extraction is performed 2-4 times.

7. The method for obtaining a sequoyitol-containing extract according to claim 1, **characterized in that** the solvent used in the two-phase extraction is a water-immiscible organic solvent and water.

8. The method for obtaining a sequoyitol-containing extract according to claim 7, **characterized in that** the water-immiscible organic solvent is selected from the group consisting of ethyl acetate, chloroform, dichloromethane, ethyl ether, petroleum ether, n-butanol, and any combination thereof.

9. The method for obtaining a sequoyitol-containing extract according to claim 7, **characterized in that** the ratio of the water-immiscible organic solvent to water is 1:0.5 to 10 (v/v).

10. The method for obtaining a sequoyitol-containing extract according to claim 1, **characterized in that** the column chromatography is macroporus resin column chromatography, sephadex column chromatography, modified sephadex column chromatography, cellulose column chromatography, activated charcoal column chromatography or ion-exchange resin column chromatography.

11. The method for obtaining a sequoyitol-containing extract according to claim 1, **characterized in that** a purification step is performed after the filtration and before the drying.

12. The method for obtaining a sequoyitol-containing extract according to claim 11, **characterized in that** the purification is column chromatography.

13. The extract obtainable by the method for obtaining a sequoyitol-containing extract according to any one of claims 1-12.

14. Use of the extract according to claim 13 or pharmaceutically acceptable salts thereof for the preparation of medicaments for treating diabetes and its complications.

15. The use according to claim 14, **characterized in that** the pharmaceutically acceptable salts include the following salts of sequoyitol: citrate, tartrate, acetate, lactate, sodium salt, potassium salt, calcium salt, ammonium salt, magnesium salt, ferrum salt, zinc salt, aluminum salt, and any combination thereof.

16. The use according to claim 14, **characterized in that** the complications include ocular disorders, kidney disorders, neuropathy, coronary heart disease, cerebrovascular disorders, or peripheral vascular disorders.
